# EUROPEAN PATENT SPECIFICATION

(11) **EP 2 218 709 B1**
(45) Date of publication and mention of the grant of the patent: **19.03.2014**
(21) Application number: 08856520.5
(22) Date of filing: 02.12.2008
(51) Int. Cl.: C07C 68/00, C07C 69/96, C07D 317/36, C07D 317/38, C07D 319/06, C07B 61/00

(54) **METHOD FOR PRODUCING CARBONATE COMPOUND**
VERFAHREN ZUR HERSTELLUNG EINER CARBONATVERBINDUNG
PROCÉDÉ DE PRODUCTION D'UN COMPOSÉ DE CARBONATE

(30) Priority: 03.12.2007 JP 2007312655; 13.12.2007 JP 2007321773; 13.08.2008 JP 2008208726
(43) Date of publication of application: 18.08.2010
(73) Proprietor: Asahi Glass Company, Limited, Chiyoda-ku Tokyo 100-8405 (JP)
(72) Inventor: OKAMOTO, Hidekazu, Tokyo 100-8405 (JP); TAJIMA, Kouhei, Tokyo 100-8405 (JP); OKAZOE, Takashi, Tokyo 100-8405 (JP)
(74) Representative: Müller-Boré & Partner Patentanwälte PartG mbB
(86) International application number: PCT/JP2008/071903
(87) International publication number: WO 2009/072501

(56) References cited:
- RU-C1- 2 309 935
- US-A- 4 353 831
- US-A- 4 786 741
- US-A- 5 091 543
- HONG ZHU ET AL: "SYNTHESIS OF PROPYLENE CARBONATE AND SOME DIALKYL CARBONATES IN THEPRESENCE OF BIFUNCTIONAL CATALYST COMPOSITIONS" POLYMERS FOR ADVANCED TECHNOLOGIES, WILEY & SONS, BOGNOR REGIS, GB LNKD- DOI:10.1002/(SICI)1099-1581(199608)7:8<701 ::AID-PAT563>3.3.CO;2-F, vol. 7, no. 8, 1 August 1996 (1996-08-01), pages 701-703, XP000623427 ISSN: 1042-7147

## Description

### TECHNICAL FIELD

The present invention relates to a novel process for producing a carbonate compound.

### BACKGROUND ART

As processes for producing a carbonate compound, the following processes are known (US 4 353 831).
(1) A process for producing a cyclic carbonate by reacting carbon dioxide gas with an alkene oxide in the presence of a catalyst (see, e.g., Patent Document 1).
(2) A process for producing a dialkyl carbonate or a cyclic carbonate by reacting phosgene (COCl₂) with an alcohol (see, e.g., Patent Document 2).
(3) A process for producing a carbonate compound by an ester exchange reaction of a cyclic carbonate or dimethyl carbonate with an alcohol in the presence of an ester exchange reaction catalyst (see, e.g., Non-Patent Document 1).
(4) A process for producing a carbonate compound by reacting methyl chloroformate with an alcohol (see, e.g., Patent Document 2).

However, the process (1) involves a problem that only cyclic carbonates are produced and various carbonates cannot be selectively produced.

The process (2) involves problems that production facilities are corroded with hydrogen chloride produced as a by-product; phosgene has toxicity; and the like.

Since the process (3) is an equilibrium reaction, it involves problems that a large excess of an alcohol should be used for improving the yield of the objective product; it is difficult to separate and remove an asymmetrical carbonate compound produced as a by-product; and the like.

The process (4) involves problems that production facilities are corroded with hydrogen chloride produced as a by-product; and the like.

Also, as examples of reacting hexachloroacetone with an alcohol, the following examples have been reported.
(5) An example of synthesis of trichloroacetate by the reaction of hexachloroacetone with methanol (Non-Patent Document 2).
(6) An example wherein the formation of di(2-methyl-2-propen-1-yl) carbonate is confirmed by the reaction of hexachloroacetone with 2-methyl-2-propen-1-ol at room temperature or a lower temperature (Non-Patent Document 3).
(7) An example wherein a cyclic alkylene carbonate and chloroform are formed by the reaction of a vicinal diol compound (propylene glycol or the like) with hexachloroacetone in the presence of a base catalyst (a salt of a strong base with a weak acid) (Patent Document 3).
(8) An example wherein a cyclic alkylene carbonate and chloroform are formed by the reaction of a vicinal diol compound (propylene glycol or the like) with hexachloroacetone using a Group 2 or 3 metal hydrosilicate catalyst (Patent Document 4).

However, in the example (5), the formation of a carbonate compound has not been reported.

In the example (6), a small amount of metal sodium is added during the reaction and the above carbonate is produced as a by-product in an amount of about one half of the stoichiometric amount of the metal sodium added. Concerning the reaction, it is presumed that a carbonate compound is formed through the reaction of metal sodium with a starting alcohol and the subsequent reaction of the formed sodium alkoxide with hexachloroacetone.

In the examples (7) and (8), the formation of chloroform and a cyclic carbonate compound has been reported by the reaction of hexachloroacetone with an alcohol in the presence of the catalyst. However, according to the investigation made by the present inventors, the reaction rate of the carbonate formation reaction by intramolecular cyclization is very high in the case of a diol compound having adjacent hydroxyl groups in a vicinal position and it is expected that the direct application to the reactions with other diols and monools will be difficult.

Patent Document 1: JP-A-07-206847
Patent Document 2: JP-A-60-197639
Patent Document 3: U. S. Patent No. 4353831
Patent Document 4: Russian Patent No. 2309935
Non-Patent Document 1: Journal of Catalysis, 2006, Vol. 241, No. 1, p. 34-44
Non-Patent Document 2: Analytical Chemistry, 1983, Vol. 55, No. 8, p. 1222-1225
Non-Patent Document 3: Journal of Organic Chemistry, 1979, Vol. 44, No. 3, p. 359-363

### DISCLOSURE OF THE INVENTION

### PROBLEMS TO BE SOLVED BY THE INVENTION

The invention provides a novel production process capable of selectively producing various kinds of carbonate compounds without any inhibition in high yields without using any toxic compounds such as phosgene and without producing any corrosive gas such as hydrogen chloride.

### MEANS FOR SOLVING THE PROBLEMS

The process for producing a carbonate compound of the invention is a process for producing a carbonate compound comprising reacting a compound represented by the following formula (1) with a compound having one OH group or a compound having two or more OH groups in the presence of a catalyst to obtain a compound having a carbonate bond, wherein the catalyst comprises a halogen salt:

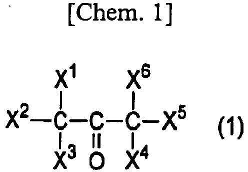

wherein X¹ to X⁶ each represents a chlorine atom.

The above halogen salt preferably comprises one or more member selected from the group consisting of halogen salts of alkali metals, halogen salts of alkali earth metals, halogen salts of ammoniums, halogen salts of quaternary ammoniums, and ion-exchange resins having a halogen salt structure.

The above halogen salt is preferably a fluoride of an alkali metal or a quaternary ammonium bromide.

In the process for producing a carbonate compound of the invention, it is preferred that the reaction is carried out in the presence of the catalyst and a promoter, wherein the promoter is a solid acid catalyst.

The solid acid catalyst preferably comprises at least one member selected from the group consisting of metal oxides having a strong acid point, heteropoly acids, and cation-exchange resins.

The metal oxides having a strong acid point preferably comprises at lest one member selected from the group consisting of cerium oxide (CeO₂/Ce₂O₃), zirconia (ZrO₂), silica-alumina (SiO₂·Al₂O₃), γ-alumina (Al₂O₃), silica-magnesia (SiO₂·MgO), silica-zirconia (SiO₂·ZrO₂), ZnO·ZrO₂, and Al₂O₃·B₂O₃.

The compound having a carbonate bond is preferably a compound represented by the following formula (31) or a compound represented by the following formula (32).

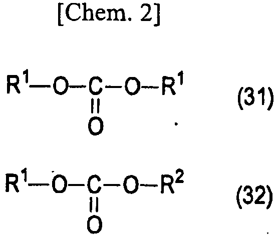

wherein R¹ and R² each represents a monovalent aliphatic hydrocarbon group or a monovalent aromatic hydrocarbon group, provided that R¹ and R² are not the same group.

The compound having a carbonate bond is preferably a cyclic carbonate compound represented by the following formula (3 a) or a linear carbonate compound represented by the following formula (3b).

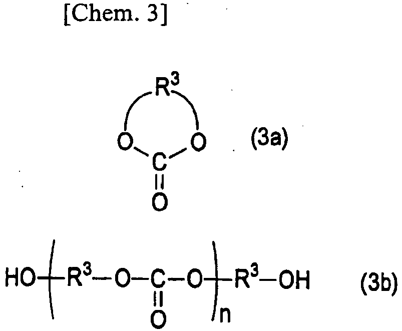

wherein R³ represents a divalent aliphatic hydrocarbon group or a divalent aromatic hydrocarbon group.

The compound having one OH group preferably comprises at least one member selected from the group consisting of methanol, ethanol, n-propanol, i-propanol, n-butanol, t-butanol, 3-oxa-n-butanol, and phenol.

The compound having two or more OH groups preferably comprises at least one member selected from the group selected from ethylene glycol, 1,2-propylene glycol, 3-methyl-1,5-pentanediol, 3-oxa-1,5-pentanediol, 1,6-hexanediol, 1,3-propanediol, 1,2-butanediol, and 1,4-butanediol.

### ADVANTAGEOUS EFFECTS OF THE INVENTION

According to the process for producing a carbonate compound of the invention, various kinds of carbonate compounds can be selectively produced without any inhibition in high yields without using any toxic compound such as phosgene and without producing any corrosive gas such as hydrogen chloride. Moreover, in addition to cyclic carbonates, oligomers or polymers of carbonates having a reactive functional group can be easily produced.

### BEST MODE FOR CARRYING OUT THE INVENTION

In the present specification, the compound represented by the formula (1) is referred to as compound (1). The compounds represented by the other formulae are also similarly referred to.

### <Carbonate compounds>

The carbonate compounds obtained by the production process of the invention are compounds having a carbonate bond (-O-C(=O)-O-).

Examples of the carbonate compounds include the compound (31), the compound (32), the compound (3a), the compound (3b), and the branched carbonate compound having two or more terminal OH groups (hereinafter referred to as branched carbonate compound).

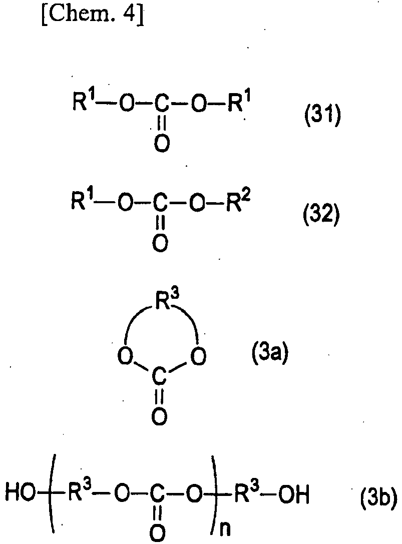

### (Compound (31))

R¹ represents a monovalent aliphatic hydrocarbon group or a monovalent aromatic hydrocarbon group. R¹'s on the left and right sides are the same.

The monovalent aliphatic hydrocarbon group may contain an etheric oxygen atom.

The monovalent aliphatic hydrocarbon group may be linear, branched, or cyclic.

R¹ may have a substituent (excluding a fluorine atom). As the substituent, a halogen atom (excluding a fluorine atom) is preferred in view of usefulness of the compound (31).

As the monovalent aliphatic hydrocarbon group, an aliphatic hydrocarbon group having 1 to 6 carbon atoms is preferred and, in view of usefulness of the compound (31), a methyl group, an ethyl group, an n-propyl group, an i-propyl group, an n-butyl group, or a t-butyl group is more preferred.

The monovalent aromatic hydrocarbon group may have a substituent of an aliphatic hydrocarbon group or an aromatic hydrocarbon group on the aromatic nuclei.

As the monovalent aromatic hydrocarbon group, an aromatic hydrocarbon group having 6 to 16 carbon atoms is preferred.

Examples of the monovalent aromatic hydrocarbon group include a phenyl group, a methylphenyl group, an ethylphenyl group and a naphthyl group, and a phenyl group is preferred in view of usefulness of the compound (31).

### (Compound (32))

R¹ and R² each represents a monovalent aliphatic hydrocarbon group or a monovalent aromatic hydrocarbon group and R¹ and R² are not the same group.

The monovalent aliphatic hydrocarbon group may contain an etheric oxygen atom.

The monovalent aliphatic hydrocarbon group may be linear, branched, or cyclic.

R¹ may have a substituent (excluding a fluorine atom). As the substituent, a halogen atom (excluding a fluorine atom) is preferred in view of usefulness of the compound (32).

As the monovalent aliphatic hydrocarbon group, an aliphatic hydrocarbon group having 1 to 6 carbon atoms, which may have an etheric oxygen atom, are preferred and, in view of usefulness of the compound (32), a methyl group, an ethyl group, an n-propyl group, an i-propyl group, an n-butyl group, a t-butyl group, a 3-oxa-n-butyl group is more preferred.

The monovalent aromatic hydrocarbon group may have a substituent of an aliphatic hydrocarbon group or an aromatic hydrocarbon group on the aromatic nucleus.

As the monovalent aromatic hydrocarbon group, an aromatic hydrocarbon group having 6 to 16 carbon atoms is preferred.

Examples of the monovalent aromatic hydrocarbon group include a phenyl group, a methylphenyl group, an ethyl group and a naphthyl group, and a phenyl group is preferred in view of usefulness of the compound (32).

The asymmetrical compound (32) is known to have a melting point lower that that of the symmetrical compound (31) and is predicted to be superior in the case where it is used as a solvent or the like.

### (Compound (3a))

The compound (3a) is a cyclic carbonate compound.

R³ represents a divalent aliphatic hydrocarbon group or a divalent aromatic hydrocarbon group.

The divalent aliphatic hydrocarbon group may contain an etheric oxygen atom.

The divalent aliphatic hydrocarbon group may be linear, branched, or cyclic.

R³ may have a substituent (excluding a fluorine atom). As the substituent, a halogen atom (excluding a fluorine atom) is preferred in view of usefulness of the compound (3a).

As R³, an aliphatic hydrocarbon group having 1 to 15 carbon atoms is preferred and, in view of usefulness of the compound (3a), -CH₂CH₂-, - CH₂CH(CH₃)-, -CH₂CH(C₂H₅)-, or -CH₂CH₂CH₂- is more preferred.

As the compound (3a), ethylene carbonate, 1,2-propylene carbonate, 1,3-propylene carbonate, or 1,2-butylene carbonate is preferred.

### (Compound (3b))

The compound (3b) is an oligomer or polymer having a reactive functional group at the terminal.

R³ represents a divalent aliphatic hydrocarbon group or a divalent aromatic hydrocarbon group. In the case where a plurality of R³'s are present in the compound (3b), R³'s may be a single kind or may be two or more kinds.

The divalent aliphatic hydrocarbon group may contain an etheric oxygen atom.

The divalent aliphatic hydrocarbon group may be linear, branched, or cyclic.

R³ may have a substituent (excluding a fluorine atom). As the substituent, a halogen atom (excluding a fluorine atom) is preferred in view of usefulness of the compound (3b).

As R³, an aliphatic hydrocarbon group having 1 to 15 carbon atoms, which may have an etheric oxygen atom, or a group represented by the following formula (4) is preferred and, in view of usefulness of the compound (3b), - CH₂CH₂CH(CH₃)CH₂CH₂-, -CH₂CH₂CH₂CH₂CH₂CH₂-, -CH₂CH₂CH₂CH₂-, - CH₂CH₂CH₂-,-CH₂CH₂(OCH₂CH₂)ₓ- (where x is an integer of 1 to 4), dipropylene glycol, tripropylene glycol, or a group represented by the following formula (4) is more preferred.

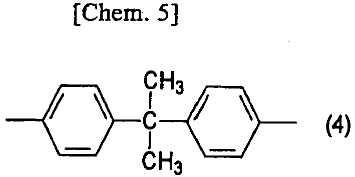

The symbol n in formula (3b) represents an integer of 1 to 1000, preferably an integer of 5 to 100, and more preferably an integer of 10 to 50. In this connection, the compound (3b) as a reaction product is usually obtained as a mixture of compounds having different n numbers.

Examples of the compound (3b) include poly(1,3-propylene carbonate), poly(1,4-butylene carbonate), poly(3-methyl-1,5-pentylene carbonate), poly(3-oxa-1,5-pentylene carbonate), poly(1,6-hexylene carbonate), -(CH₂CH₂OCH₂CH₂-O-(CO)-O)ₙ-, -(CH₂CH₂OCH₂CH₂OCH₂CH₂-O-(CO)-O)ₙ-,-(CH₂CH₂OCH₂CH₂OCH₂CH₂OCH₂CH₂-O-(CO)-O)ₙ-, -((CH(CH₃)CH₂O)_{z}-(CO)-O)ₙ-(where z is 2 or 3), and copolymers having these repeating units.

### (Branched carbonate compound)

Examples of the branched carbonate compound include branched oligomers and branched polymers, each having more than two terminal OH groups. Examples of the branched carbonate compound having more than two terminal OH groups include those having three or more terminal OH groups and mixtures of those having two terminal OH groups and those having three or more terminal OH groups. In the case of the mixtures, the number of OH groups is judged with the average value, and "more than two" represents, for example, 2.05, 2.1, or the like.

### <Process for producing carbonate compound>

The process for producing a carbonate compound of the invention is a process for producing a compound having a carbonate bond by reacting the compound (1) with a compound having one OH group or a compound having two or more OH groups in the presence of a catalyst, wherein a halogen salt is used as the catalyst.

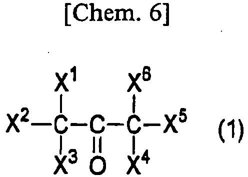

### (Compound (1))

X¹ to X³ each represents a hydrogen atom or a halogen atom, and at least one of X¹ to X³ is a halogen atom.

X⁴ to X⁶ each represents a hydrogen atom or a halogen atom, and at least one of X⁴ to X⁶ is a halogen atom.

X¹ to X⁶ are all chlorine atoms. From the viewpoint that chloroform is obtained as a by-product, they are all chlorine atoms.

Example the compound (1) include hexachloroacetone. In view of capability of simultaneous production of industrially useful chloroform in a high yield, hexachloroacetone is used.

Among the compounds (1), chloroacetones can be easily produced by the processes of chlorinating acetone as described in JP-B-60-52741 and JP-B-61-16255. Moreover, partially fluorinated compounds can be easily produced by fluorinating chloroacetones with hydrogen fluoride as described in US Patent No. 6235950.

### (Catalyst)

The halogen salt is preferably one or more member selected from the group consisting of halogen salts of alkali metals, halogen salts of alkali earth metals, halogen salts of ammoniums, halogen salts of quaternary ammoniums, and ion-exchange resins having a halogen salt structure.

In the present specification, the halogen salt means a salt of a metallic or organic cation with a halogen ion. Examples of the halogen salts of alkali metals include LiF, LiCl, LiBr, NaF, NaCl, NaBr, KF, KCl, KBr, RbF, RbCl, RbBr, CsF, CsCl, and CsBr.

Examples of the halogen salts of alkali earth metals include BeF₂, BeCl₂, BeBr₂, CaF₂, CaCl₂, CaBr₂, SrF₂, SrCl₂, and SrBr₂.

Examples of the halogen salts of ammoniums include NH₄F, NH₄Cl, and NH₄Br.

Examples of the halogen salts of quaternary ammoniums include the compound (5): 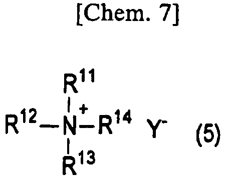 wherein R¹¹ to R¹⁴ each represents a hydrocarbon group and Y⁻ represents a halogen ion.

Examples of R¹¹ to R¹⁴ include alkyl groups, cycloalkyl groups, alkenyl groups, cycloalkenyl groups, aryl groups, alkylaryl groups and aralkyl groups, and alkyl groups, aryl groups or aralkyl groups are preferred.

The total number of the carbon atoms of R¹¹ to R¹⁴ is preferably 4 to 100 per one molecule of R¹¹R¹²R¹³R¹⁴N⁺.

R¹¹ to R¹⁴ may be the same group or may be different groups.

R¹¹ to R¹⁴ may be substituted with functional group(s) inert under reaction conditions. Although the inert functional group varies depending on the reaction conditions, examples thereof include a halogen atom, an ester group, a nitrile group, an acyl group, a carboxyl group and an alkoxyl group.

R¹¹ to R¹⁴ may be combined with each other to form a heterocyclic ring including a nitrogen-containing heterocyclic ring or the like.

R¹¹ to R¹⁴ may be a part of a polymer compound.

Examples of R¹¹R¹²R¹³R¹⁴N⁺ include a tetramethylammonium ion, a tetraethylammonium ion, a tetra-n-propylammonium ion, a tetra-n-butylammonium ion, a tri-n-octylmethylammonium ion, a cetyltrimethylammonium ion, a benzyltrimethylammonium ion, a benzyltriethylammonium ion, a cetylbenzyldimetylammonium ion, a cetylpyridinium ion, an n-dodecylpyridinium ion, a phenyltrimetylammonium ion, a phenyltriethylammonium ion, an N-benzylpicolinium ion, a pentamethonium ion, and a hexamethonium ion.

Examples of Y⁻ include a chlorine ion, a fluorine ion, a bromine ion and an iodine ion, and a chlorine ion, a fluorine ion or a bromine ion is preferred.

As the compound (5), in view of versatility and reactivity of the compound (5), a combination of the following R¹¹R¹²R¹³R¹⁴N⁺ and the following Y⁻ is preferred.

R¹¹R¹²R¹³R¹⁴N⁺: a tetramethylammonium ion, a tetraethylammonium ion, a tetra-n-propylammonium ion, a tetra-n-butylammonium ion, or a tri-n-octylmethylammonium ion.

Y⁻: a fluorine ion, a chlorine ion, or a bromine ion.

The ion-exchange resins having a halogen salt structure include anion type ion-exchange resins having a halogen ion as an anion. Examples of commercially available products include DIAION (registered trademark) series (manufactured by Mitsubishi Chemical Corporation), Amberlite (registered trademark) series (manufactured by Rohm and Haas Company), and Amberlyst (registered trademark) series (manufactured by Rohm and Haas Company).

As the halogen salt, in view of reactivity and utilization in an industrial scale, a fluoride of an alkali metal (NaF, KF, or the like) or a quaternary ammonium bromide is preferred.

The halogen salt may be supported on a metal oxide or a composite oxide. Examples of the compound include soda lime.

### (Promoter).

In the process for producing a carbonate compound of the invention, it is preferred to obtain the above compound having a carbonate bond in the presence of a catalyst and a promoter. Using the promoter, catalyst activity can be improved.

As the promoter, a solid acid catalyst is used.

The solid acid catalyst is preferably at least one member selected from the group consisting of metal oxides having a strong acid point, heteropoly acids, and cation-exchange resins.

Examples of the metal oxides having a strong acid point include SiO₂·Al₂O₃, SiO₂·MgO, SiO₂·ZrO₂, Al₂O₃·B₂O₃, Al₂O₃, ZrO₂, ZnO·ZrO₂, CeO₂, Ce₂O₃, various zeolites, and the like. In view of acid strength and reaction selectivity, at least one member selected from the group consisting of cerium oxide (CeO₂/Ce₂O₃), silica-alumina (SiO₂·Al₂O₃), γ-alumina (Al₂O₃), silica-magnesia (SiO₂·MgO), zirconia (ZrO₂), silica-zirconia (SiO₂·ZrO₂), ZnO·ZrO₂, and Al₂O₃-B₂O₃ is preferred.

### (Process for producing compound (31))

The compound (31) is produced by reacting the compound (1) with a compound (21) in the presence of a halogen salt as the catalyst.

[Chem. 8] R¹-OH (21)

Examples of the compound (21) include a monovalent aliphatic alcohol and a monovalent phenol.

As the monovalent aliphatic alcohol, in view of versatility on industrial use, a saturated aliphatic alcohol is preferred and an alkane-monool having 1 to 10 carbon atoms is more preferred.

Examples of the alkane-monool having 1 to 10 carbon atoms include methanol, ethanol, n-propanol, i-propanol, n-butanol, s-butanol, t-butanol, 1-pentanol, 2-pentanol, 2-methyl-2-butanol, 3-methyl-1-butanol, 2-ethylbutanol, tetrahydrofurfuryl alcohol, neopentyl alcohol, n-octanol, furfuryl alcohol, 3-pentanol, 2-methyl-1-butanol, 3-methyl-2-butanol, 4-methyl-2-pentanol, allyl alcohol, 1-hexanol, cyclohexanol, ethylene glycol monoethyl ether, ethylene glycol monobutyl ether, ethylene glycol monomethyl ether (3-oxa-n-butanol), ethylene glycol monomethoxymethyl ether, ethylene chlorohydrin, diethylene glycol monoethyl ether, diethylene glycol monomethyl ether, propylene glycol monoethyl ether, propylene glycol monomethyl ether, propylene chlorohydrin, and the like.

The monovalent aliphatic alcohol is more preferably an alkane-monool having 1 to 6 carbon atoms in view of usefulness of the compound (31). Specifically, methanol, ethanol, n-propanol, i-propanol, n-butanol, t-butanol, or 3-oxa-n-butanol is more preferred.

Examples of the monovalent phenol include phenol, ethylphenol, octylphenol, dimethylphenol, o-methoxyphenol, cresol, hydroxybiphenyl, p-cumylphenol, naphthol, and benzylphenol. In view of usefulness of the compound (31), phenol is preferred.

The ratio of the first charged molar amount of the compound (21) to the first charged molar amount of the compound (1) (compound (21)/compound (1)) is preferably more than 1, more preferably 1.5 or more, and particularly preferably 2 or more in view of improving the yield of the compound (31). By regulating the ratio to more than 1, the reaction equilibrium shifts to the compound (31) side, thereby the reaction yield being improved.

The amount of the catalyst is variously selected depending on the catalyst, but is preferably 0.01 to 30% by mass and, in consideration of reactivity and a catalyst removal step after the reaction, is more preferably 0.1 to 10% by mass based on the substrate.

The amount of the promoter is variously selected depending on the promoter, but is preferably 0.01 to 30% by mass and, in consideration of reactivity and a promoter removal step after the reaction, is more preferably 0.1 to 10% by mass based on the substrate.

Since the compound (21) mostly has a low compatibility with the compound (1), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the compound (21), the compound (31), and by-products by distillation after the reaction, it is preferred to use a solvent having a boiling point different from that of each of these compounds or to use the compound (31) as the solvent.

As the solvent, carbonate compounds different in boiling point, the compound (31), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme, and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

In the invention, at least a part of the reaction between the compound (1) and the compound (21) is preferably carried out at a reaction temperature of 40 to 200°C.

When the reaction temperature is lower than 40°C, the yield of the carbonate compound is extremely low. When the reaction temperature exceeds 200°C, decrease in yield owing to the decomposition of the compound (1) to be used as a starting material becomes remarkable. When the reaction temperature falls within the above range, the carbonate compound can be produced in high yields at a reaction rate capable of industrial use.

The reaction temperature is more preferably 40 to 160°C, more preferably 50 to 150°C, and particularly preferably 60 to 140°C.

The efficiency of the reaction can be improved by carrying out the reaction at different reaction temperatures at the early reaction stage and at the later reaction stage. This is because the substitution reactions of the two functional groups in the compound (1) proceeds stepwise and the reaction rate of the first step substitution reaction is high but the reaction rate of the second substitution reaction is comparatively low. Since the first step substitution reaction easily proceeds at a relatively low temperature of about 0 to 100°C and is a reaction with severe heat generation for a while, the reaction is preferably allowed to proceed at a relatively low temperature at the early reaction stage. The second step substitution reaction is carried out at a relatively high temperature of about 50 to 200°C in view of the reaction rate.

The reaction pressure is usually atmospheric pressure. Depending on the vapor pressure of the compound (21) at the reaction temperature, it is preferred to apply pressure.

In the present reaction, CHX¹X²X³ and/or CHX⁴X⁵X⁶ (chloroform and the like), which are halogenated methanes having a low boiling point, are formed as the reaction proceeds. Accordingly, in order to improve the reaction yield by shifting the reaction equilibrium to the compound (31) side and to complete the reaction stoichiometrically, it is preferred to carry out the reaction with removing the formed CHX¹X²X³ and/or CHX⁴X⁵X⁶ from the reaction system by distillation.

As a method for removing halogenated methanes by distillation, a reaction distillation mode utilizing the fact that the halogenated methanes each has a low boiling point as compared with the compound (21) and the compound (31) is preferred from the viewpoint of easy implementation.

### (Process for producing compound (32))

The compound (32) is preferably produced by reacting the compound (1) with the compound (21) in the presence of a halogen salt as the catalyst to obtain a compound (11a) and/or a compound (11b) (hereinafter the compound (11a) and the compound (11b) are collectively referred to as compound (11)) and successively reacting the compound (11) with a compound (22).

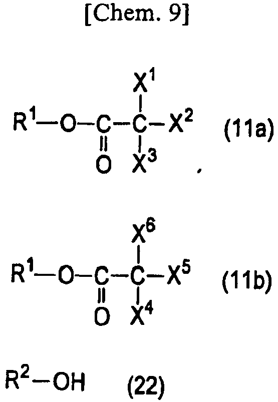

Moreover, the compound (1), the compound (21), and the compound (22) may be reacted at the same time. In that case, the compound (32), the compound (31), and the compound (33) are obtained as a mixture.

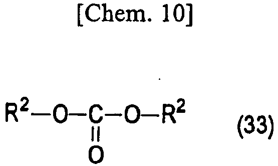

Examples of the compound (22) include the above-mentioned monovalent aliphatic alcohols and monovalent phenols. However, as the compound (22), an alcohol different from the compound (21) is used.

The monovalent aliphatic alcohol is preferably an alkane-monool having 1 to 6 carbon atoms, more preferably an alkane-monool having 1 to 4 carbon atoms, which may have an etheric oxygen atom, in view of usefulness of the compound (32).

As the alkane-monool having 1 to 6 carbon atoms, methanol, ethanol, n-propanol, i-propanol, n-butanol, t-butanol, or 3-oxa-n-butanol is preferred.

As the monovalent phenol, in view of usefulness of the compound (32), phenol is preferred.

The ratio of the first charged molar amounts of the compound (21) and the compound (22) to the first charged molar amount of the compound (1) ((compound (21)+ compound (22))/compound (1)) is preferably more than 1, more preferably 1.5 or more, and particularly preferably 2 or more.

Moreover, in view of improving the yield of the compound (32), it is preferred that the compound (21) is reacted with the compound (1) in a ratio of 1 molar equivalent or less to the latter compound to selectively form the compound (11) and then the compound (22) is reacted with the compound (11) in a ratio of 1 to 2 molar equivalents to the latter compound. When the amount of the compound (22) is less than 1 molar equivalent, the yield of the objective compound (32) decreases. When the amount is more than 2 molar equivalents, the compound (33) is formed by the ester exchange reaction between the formed compound (32) and the compound (22), so that the yield of the objective compound (32) decreases.

The amount of the catalyst is variously selected depending on the catalyst, but is preferably 0.01 to 30% by mass and, in consideration of the reaction activity and a catalyst removal step after the reaction, is more preferably 0.1 to 10% by mass based on the substrate.

The amount of the promoter is variously selected depending on the promoter, but is preferably 0.01 to 30% by mass and, in consideration of the reaction activity and a promoter removal step after the reaction, is more preferably 0.1 to 10% by mass based on the substrate.

Since the compound (21) and the compound (22) mostly have a low compatibility with the compound (1) and the compound (11), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the compound (11), the compound (21), the compound (22), the compound (32), and by-products by distillation after the reaction, it is preferred to use a solvent having a boiling point different from that of each of these compounds or to use the compound (32) as the solvent.

As the solvent, carbonate compounds different in boiling point, the compound (32), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme, and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

In the invention, at least a part of the reaction between the compound (1) and the compound (21) and/or the compound (22) is preferably carried out at a reaction temperature of 40 to 200°C.

When the reaction temperature is lower than 40°C, the yield of the carbonate compound is extremely low. When the reaction temperature exceeds 200°C, decrease in yield owing to the decomposition of the compound (1) to be used as a starting material becomes remarkable. When the reaction temperature falls within the above range, the carbonate compound can be produced in high yields at a reaction rate capable of industrial use.

The reaction temperature is more preferably 40 to 160°C, more preferably 50 to 150°C, and particularly preferably 60 to 140°C.

The efficiency of the reaction can be improved by carrying out the reaction at different reaction temperatures at the early reaction stage and at the later reaction stage. Namely, the reaction of forming the compound (11) by reacting the compound (21) with the compound (1) is preferably at a reaction temperature of 40°C or lower in view of improving the yield of the compound (11). The reaction can be carried out at a temperature higher than 40°C but since the reaction is too vigorous, by-products may be increased or the compound (31) that is a disubstituted product may be formed, thereby the yield of the objective product being lowered in some cases. In the case where the compound (1) is reacted with the compound (21) at the reaction temperature of 40°C or lower, the compound (11) can be selectively synthesized even when the compound (21) is reacted with the compound (1) in a ratio of 1 equivalent or more to the latter compound. However, unless the reaction is carried out after unreacted compound (21) is removed from the reaction system before the next compound (22) is reacted, the lowering of the yield of the objective compound (32) may be caused through the production of the compound (31) as a by-product.

The reaction between the compound (11) and the compound (22) is preferably carried out at a reaction temperature of 40 to 200°C, and more preferably carried out at a reaction temperature of 50 to 200°C.

Thus, since the difference between the reaction rate of the first step and the reaction rate of the second step is large, there are advantages that the compound (11) as an intermediate can be easily synthesized and isolated and the asymmetrical compound (22), which is hitherto hardly synthesized, can be selectively synthesized utilizing the difference between the reaction rates.

The reaction pressure is usually atmospheric pressure. Depending on the vapor pressure of the compound (21) and the compound (22) at the reaction temperature, it is preferred to apply pressure.

In the present reaction, CHX¹X²X³ and/or CHX⁴X⁵X⁶ (chloroform and the like), which are halogenated methanes having a low boiling point, are formed as the reaction proceeds. Accordingly, in order to improve the reaction yield by shifting the reaction equilibrium to the compound (32) side and to complete the reaction stoichiometrically, it is preferred to carry out the reaction with removing the formed CHX¹X²X³ and/or CHX⁴X⁵X⁶ from the reaction system by distillation.

As a method for removing halogenated methanes by distillation, a reaction distillation mode utilizing the fact that the halogenated methanes each has a low boiling point as compared with the compound (21), the compound (11), the compound (22) and the compound (32) is preferred from the viewpoint of easy implementation.

### (Process for producing compound (3a), compound (3b))

The compound (3a) and the compound (3b) are produced by reacting the compound (1) with a compound (23) in the presence of a halogen salt as the catalyst.

[Chem. 11] HO-R³-OH (23)

Examples of the compound (23) include divalent aliphatic alcohols and divalent phenols.

Examples of the divalent aliphatic alcohols include, in view of versatility on industrial use, ethylene glycol, diethylene glycol (3-oxa-1,5-pentanediol), triethylene glycol, tetraethylene glycol, dipropylene glycol, tripropylene glycol, 3-chloro-1,2-propanediol, 2-chloro-1,3-propanediol, cyclohexanediol, 1,2-propylene glycol, 1,3-propanediol, 1,2-butanediol, 1,3-butanediol, 1,4-butanediol, 1,4-butenediol, 2-methyl-2,4-pentanediol (hexylene glycol), 3-methyl-1,5-pentanediol, 1,5-pentanediol, and 1,6-hexanediol.

The divalent aliphatic alcohol is, in view of usefulness of the compound (3a) and the compound (3b), preferably ethylene glycol, 1,2-propylene glycol, 3-methyl-1,5-pentanediol, 1,6-hexanediol, 1,3-propanediol, and 1,4-butanediol, more preferably 1,2-propylene glycol (HO-CH₂CH(CH₃)-OH), ethylene glycol (HO-CH₂CH₂-OH), or 3-oxa-1,5-pentanediol (HO-CH₂CH₂OCH₂CH₂-OH).

Examples of the divalent phenols include resorcinol, catechol, hydroquinone, 2,2-bis(4-hydroxyphenyl)propane [bisphenol A], 4,4'-dihydroxybiphenyl, and dihydroxynaphthalene. In view of easy availability of the starting material, bisphenol A is preferred.

In the case where the objective product is the compound (3 a), with regard to the ratio of the substrates (starting materials), the ratio of the compound (23) is preferably 0.1 to 10 molar equivalents to the compound (1) and, in view of the reaction efficiency and the yield, is more preferably 0.5 to 2 molar equivalents.

In the case where the objective product is the compound (3b), the ratio of the substrates varies depending on the molecular weight of the compound (3b) but the ratio of the compound (23) is preferably 0.5 to 2 molar equivalents to the compound (1) and, in view of the reaction efficiency and the yield, is more preferably 0.75 to 1.5 molar equivalents.

The amount of the catalyst is variously selected depending on the catalyst, but is preferably 0.01 to 30% by mass and, in consideration of reactivity and a catalyst removal step after the reaction, is more preferably 0.1 to 10% by mass based on the substrate.

The amount of the promoter is variously selected depending on the promoter, but is preferably 0.01 to 30% by mass and, in consideration of reactivity and a promoter removal step after the reaction, is more preferably 0.1 to 10% by mass based on the substrate.

Since the compound (23) mostly has a low compatibility with the compound (1), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the compound (23), the compound (3a), the compound (3b), and by-products by distillation after the reaction, it is preferred to use a solvent having a boiling point different from that of each of these compounds or to use the compound (3a) as the solvent.

As the solvent, carbonate compounds different in boiling point, the compound (3 a), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme, and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

The reaction temperature varies depending on the substrates, catalysts, and the like and is usually 0 to 200°C.

The efficiency of the reaction can be improved by carrying out the reaction at different reaction temperatures at the early reaction stage and at the later reaction stage. This is because the substitution reactions of the two functional groups in the compound (1) proceeds stepwise and the reaction rate of the first step substitution reaction is high but the reaction rate of the second substitution reaction is comparatively low. Since the first step substitution reaction easily proceeds at a relatively low temperature of about 0 to 100°C and is a reaction with severe heat generation for a while, the reaction is preferably allowed to proceed at a relatively low temperature at the early reaction stage. The second step substitution reaction is carried out at a relatively high temperature of about 50 to 200°C in view of the reaction rate.

In this connection, in the case where the objective product has a stable 5-membered ring structure, such as ethylene carbonate or propylene carbonate, since a stabilization effect by cyclization is large, the reaction of the second step also proceeds at a very high reaction rate and the reaction is completed within a short period of time even at a relatively low temperature of 0 to 80°C.

The reaction pressure is usually atmospheric pressure. Depending on the vapor pressure of the compound (23) at the reaction temperature, it is preferred to apply pressure.

In the present reaction, CHX¹X²X³ and/or CHX⁴X⁵X⁶ (chloroform and the like), which are halogenated methanes having a low boiling point, are formed as the reaction proceeds. Accordingly, in order to improve the reaction yield by shifting the reaction equilibrium to the compound (3a) and compound (3b) side and to complete the reaction stoichiometrically, it is preferred to carry out the reaction with removing the formed CHX¹X²X³ and/or CHX⁴X⁵X⁶ from the reaction system by distillation.

As a method for removing halogenated methanes by distillation, a reaction distillation mode utilizing the fact that the halogenated methanes each has a low boiling point as compared with the compound (23), the compound (3a) and the compound (3b) is preferred from the viewpoint of easy implementation.

### (Process for producing branched carbonate compound)

The branched carbonate compound is produced by reacting the compound (1) with a compound having more than two OH groups in the presence of a halogen salt as the catalyst.

Examples of the compound having more than two OH groups include trivalent or higher valent aliphatic alcohols, trivalent or higher valent phenols, and mixtures of them and the above compound having two OH groups. In the case of the mixtures, the average value of the terminal OH groups is taken as the number of OH groups.

Examples of the trivalent or higher valent aliphatic alcohols include, in view of versatility on industrial use, glycerin, diglycerin, polyglycerin, trimethylolpropane, 1,2,6-hexanetriol, pentaerythritol, tetramethylolcyclohexane, methylglycoside, sorbitol, mannitol, dulcitol, and sucrose.

Examples of the trivalent or higher valent phenols include fluoroglycinol, and condensates of phenols.

Examples of the condensates of phenols include resol-type initial condensates wherein phenols are condensed and combined with excess of aldehydes in the presence of an alkali catalyst; benzylic-type initial condensates which are produced by the reaction in a non-aqueous system at the time when the resol-type initial condensates are synthesized; and novolak-type initial condensates wherein excess of phenols are reacted with formaldehydes in the presence of an acid catalyst. The molecular weight of the initial condensates is preferably about 200 to 10000.

The ratio of the substrates varies depending on the molecular weight of the branched carbonate compound but the ratio of the compound having more than two OH groups is preferably 0.5 to 2 molar equivalents to the compound (1) and more preferably 0.75 to 1.5 molar equivalents.

The amount of the catalyst is variously selected depending on the catalyst, but is preferably 0.01 to 30% by mass and, in consideration of reactivity and a catalyst removal step after the reaction, is more preferably 0.1 to 10% by mass based on the substrate.

The amount of the promoter is variously selected depending on the promoter, but is preferably 0.01 to 30% by mass and, in consideration of reactivity and a promoter removal step after the reaction, is more preferably 0.1 to 10% by mass based on the substrate.

Since the compound having more than two OH groups mostly has a low compatibility with the compound (1), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the compound having more than two OH groups, the branched carbonate carbonate compound, and by-products by distillation after the reaction, it is preferred to use a solvent having a boiling point different from that of each of these compounds or to use the compound (3a) as the solvent.

As the solvent, carbonate compounds different in boiling point, the compound (3a), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme, and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

The reaction temperature varies depending on the substrates, catalysts, and the like and is usually 0 to 200°C.

The efficiency of the reaction can be improved by carrying out the reaction at different reaction temperatures at the early reaction stage and at the later reaction stage. This is because the substitution reactions of the two functional groups in the compound (1) proceeds stepwise and the reaction rate of the first step substitution reaction is high but the reaction rate of the second substitution reaction is comparatively low. Since the first step substitution reaction easily proceeds at a relatively low temperature of about 0 to 100°C and is a reaction with severe heat generation for a while, the reaction is preferably allowed to proceed at a relatively low temperature at the early reaction stage. The second step substitution reaction is carried out at a relatively high temperature of about 50 to 200°C in view of the reaction rate.

The reaction pressure is usually atmospheric pressure. Depending on the vapor pressure of the compound having more than two OH groups at the reaction temperature, it is preferred to apply pressure.

In the present reaction, CHX¹X²X³ and/or CHX⁴X⁵X⁶ (chloroform and the like), which are halogenated methanes having a low boiling point, are formed as the reaction proceeds. Accordingly, in order to improve the reaction yield by shifting the reaction equilibrium to the branched carbonate compound side and to complete the reaction stoichiometrically, it is preferred to carry out the reaction with removing the formed CHX¹X²X³ and/or CHX⁴X⁵X⁶ from the reaction system by distillation.

As a method for removing the halogenated methanes by distillation, a reaction distillation mode utilizing the fact that the halogenated methanes each has a low boiling point as compared with the compound having more than two OH groups and the branched carbonate compound is preferred from the viewpoint of easy implementation.

Since the process for producing a carbonate compound of the invention as described in the above is a process wherein the compound (1) is reacted with the compound having one OH group in the presence of the halogen salt that is a catalyst to obtain a carbonate compound, a symmetrical dialkyl carbonate or diaryl carbonate or an asymmetrical dialkyl carbonate or diaryl carbonate can be selectively prepared without any inhibition in high yields by suitably changing the compound having one OH group in one reaction process.

Moreover, since the process for producing a carbonate compound of the invention is a process wherein the compound (1) is reacted with the compound having two or more OH groups in the presence of the halogen salt that is a catalyst to obtain a carbonate compound, a cyclic carbonate or a polycarbonate can be selectively prepared without any inhibition in high yields by suitably changing the compound having two or more OH groups in one reaction process.

Furthermore, since the by-product is an organic compound having a low boiling point, such as chloroform, a production process can be simplified, for example, by-products can be easily removed from the reaction system, unlike other methods such as a method using phosgene.

Moreover, by changing the compound (I) to hexachloroacetone, industrially useful chloroform can be simultaneously produced.

Furthermore, by the use of a partially fluorinated compound as the compound (I), industrially useful dichlorofluoromethane (R21), chlorodifluoromethane (R22), or the like can be simultaneously produced.

### EXAMPLES

The present invention will be illustrated in greater detail with reference to the following Examples, but the invention should not be construed as being limited thereby.

Examples 1 to 16 are Invention Examples and Examples 17 and 18 are Comparative Examples.

### (Gas chromatograph)

The analysis on a chromatograph (hereinafter referred to as GC) was performed using a 6890 series manufactured by Agilent Company.

### (Molecular weight)

The measurement of number-average molecular weight (hereinafter referred to as Mn) and weight-average molecular weight (hereinafter referred to as Mw) was performed using a gel permeation chromatograph (HLC-8220 GPC manufactured by Tosoh Corporation) (hereinafter referred to as GPC). Mn and Mw are molecular weights based on polystyrene standards.

### Example I

After 262 g (0.99 mol) of hexachloroacetone, 150 g (1.97 mol) of 1,2-propylene glycol, and 4 g of tetrabutylammonium bromide (hereinafter referred to as TBAB) were charged into a 500 mL glass reactor fitted with a stirrer, a reflux condenser at 20°C, and a distillation line, the temperature was gradually elevated under stirring and a reaction was carried out at an inner temperature of 120°C. While chloroform formed by the reaction was removed by distillation through the distillation line, the reaction was carried out for 10 hours. After the reaction was completed, fractions distilled from the distillation line and a reaction crude liquid present in the reactor were recovered to obtain 407.8 g of a recovered crude liquid (recovery rate: 98%). As a result of analysis on GC of an organic component recovered by simple distillation of the recovered crude liquid under vacuum, it was confirmed that compounds shown in Table 1 were formed in yields shown in Table 1.

From the results shown in Table 1, the conversion rate of hexachloroacetone was 100%, the yield of 1,2-propylene carbonate based on hexachloroacetone was 97%, and the yield of chloroform was 96%.

**[Table 1]**

| Compound | GC Composition (% by mass) | Yield |
|---|---|---|
| Hexachloroacetone | 0% | 0 g |
| 1,2-propylene glycol | 19.2% | 77.5 g |
| Chloroform | 56.0% | 226 g |
| Carbon tetrachloride | 0.3% | 1.2 g |
| 1,2-Propylene carbonate | 24.3% | 98 g |
| Others | 0.2% | 0.9 g |

### Example 2

A reaction was carried out in the same manner as in Example 1 except that the amount of 1,2-propylene glycol was changed to 75 g (0.99 mol). After the reaction was completed, fractions distilled from the distillation line and a reaction crude liquid present in the reactor were recovered to obtain 328.6 g of a recovered crude liquid (recovery rate: 96.36%). As a result of analysis on GC of an organic component recovered by simple distillation of the recovered crude liquid under vacuum, it was confirmed that compounds shown in Table 2 were formed in yields shown in Table 2.

From the results shown in Table 2, the conversion rate of hexachloroacetone was 100%, the yield of 1,2-propylene carbonate based on hexachloroacetone was 94%, and the yield of chloroform was 95%.

**[Table 2]**

| Compound | GC Composition (% by mass) | Yield |
|---|---|---|
| Hexachloroacetone | 0% | 0 g |
| 1,2-propylene glycol | 0% | 0 g |
| Chloroform | 69.3% | 225 g |
| Carbon tetrachloride | 0.2% | 0.7 g |
| 1,2-Propylene carbonate | 29.3% | 95 g |
| Others | 1.2% | 3.9 g |

### Example 3

A reaction was carried out in the same manner as in Example 1 except that the amount of 1,2-propylene glycol was changed to 100 g (1.32 mol). After the reaction was completed, fractions distilled from the distillation line and a reaction crude liquid present in the reactor were recovered to obtain 350.2 g of a recovered crude liquid (recovery rate: 95.67%). As a result of analysis on GC of an organic component recovered by simple distillation of the recovered crude liquid under vacuum, it was confirmed that compounds shown in Table 3 were formed in yields shown in Table 3.

From the results shown in Table 3, the conversion rate of hexachloroacetone was 100%, the yield of 1,2-propylene carbonate based on hexachloroacetone was 97%, and the yield of chloroform was 94%.

**[Table 3]**

| Compound | GC Composition (% by mass) | Yield |
|---|---|---|
| Hexachloroacetone | 0% | 0 g |
| 1,2-Propylene glycol | 6.9% | 23.9 g |
| Chloroform | 64.4% | 223 g |
| Carbon tetrachloride | 0.3% | 1.1 g |
| 1,2-Propylene carbonate | 28.3% | 98 g |
| Others | 0.1% | 0.2 g |

### Example 4

After 262 g (0.99 mol) of hexachloroacetone, 100 g (1.32 mol) of 1,2-propylene glycol, and 4 g of KF (potassium fluoride) were charged into a reactor similar to that of Example 1, a reaction was carried out under stirring at an inner temperature of 30°C for 2 hours. After the reaction was completed, 364.4 g of a reaction crude liquid present in the reactor were recovered (recovery rate: 99.56%). As a result of analysis on GC of an organic component recovered by simple distillation of the recovered crude liquid under vacuum, it was confirmed that compounds shown in Table 4 were formed in yields shown in Table 4.

From the results shown in Table 4, the conversion rate of hexachloroacetone was 100%, the yield of 1,2-propylene carbonate based on hexachloroacetone was 99%, and the yield of chloroform was 98%.

**[Table 4]**

| Compound | GC Composition (% by mass) | Yield |
|---|---|---|
| Hexachloroacetone | 0% | 0 g |
| 1,2-Propylene glycol | 6.7% | 24 g |
| Chloroform | 64.7% | 233 g |
| Carbon tetrachloride | 0.3% | 1.0 g |
| 1,2-Propylene carbonate | 27.7% | 100 g |
| Others | 0.6% | 2.4 g |

### Example 5

A reaction was carried out in the same manner as in Example 2 except that the amount of TBAB was changed to 0.85 g. After the reaction was completed, fractions distilled from the distillation line and a reaction crude liquid present in the reactor were recovered to obtain 333.5 g of a recovered crude liquid (recovery rate: 98.7%). As a result of analysis on GC of an organic component recovered by simple distillation of the recovered crude liquid under vacuum, it was confirmed that compounds shown in Table 5 were formed in yields shown in Table 5.

From the results shown in Table 5, the conversion rate of hexachloroacetone was 99%, the yield of 1,2-propylene carbonate based on hexachloroacetone was 97%, and the yield of chloroform was 97%.

**[Table 5]**

| Compound | GC Composition (% by mass) | Yield |
|---|---|---|
| Hexachloroacetone | 0.6% | 2 g |
| 1,2-Propylene glycol | 0.15% | 0.5 g |
| Chloroform | 68.8% | 229 g |
| Carbon tetrachloride | 0.2% | 0.7 g |
| 1,2-Propylene carbonate | 29.4% | 97.8 g |
| Others | 0.85% | 2.7 g |

### Example 6

After 262 g (0.99 mol) of hexachloroacetone, 61.4 g (0.99 mol) of ethylene glycol, and 4 g of KF were charged into a reactor similar to that of Example 1, the temperature was gradually elevated under stirring and a reaction was carried out at an inner temperature of 50°C. While chloroform formed by the reaction was removed by distillation through the distillation line, the reaction was carried out for 30 minutes. After the reaction was completed, fractions distilled from the distillation line and a reaction crude liquid present in the reactor were recovered to obtain 326.2 g of a recovered crude liquid (recovery rate: 99.6%). As a result of analysis on GC of an organic component recovered by simple distillation of the recovered crude liquid under vacuum, it was confirmed that compounds shown in Table 6 were formed in yields shown in Table 6.

From the results shown in Table 6, the conversion rate of hexachloroacetone was 100%, the yield of ethylene carbonate based on hexachloroacetone was 99%, and the yield of chloroform was 99%.

**[Table 6]**

| Compound | GC Composition (% by mass) | Yield |
|---|---|---|
| Hexachloroacetone | 0% | 0 g |
| Ethylene glycol | 0% | 0 g |
| Chloroform | 73.0% | 235.1 g |
| Carbon tetrachloride | 0.03% | 0.1 g |
| Ethylene carbonate | 26.8% | 86.5 g |
| Others | 0.17% | 0.5 g |

### Example 7

After 262 g (0.99 mol) of hexachloroacetone, 75.2 g (0.99 mol) of 1,3-propanediol, and 4 g of KF were charged into a reactor similar to that of Example 1, the temperature was gradually elevated under stirring and a reaction was carried out at an inner temperature of 120°C. While chloroform formed by the reaction was removed by distillation through the distillation line, the reaction was carried out for 10 hours. After the reaction was completed, fractions distilled from the distillation line and a reaction crude liquid present in the reactor were recovered to obtain 327.6 g of a recovered crude liquid (recovery rate: 96.0%). As a result of analysis on GC of an organic component recovered by simple distillation of the recovered crude liquid under vacuum, it was confirmed that compounds shown in Table 7 were formed in yields shown in Table 7. In the distillation under vacuum, viscous organic matter remained in the pot in addition to the catalyst.

From the results shown in Table 7, the conversion rate of hexachloroacetone was 100%, the yield of 1,3-propylene carbonate based on hexachloroacetone was 29%, and the yield of chloroform was 77%.

**[Table 7]**

| Compound | GC Composition (% by mass) | Yield |
|---|---|---|
| Hexachloroacetone | 0% | 0 g |
| 1,3-Propanediol | 0.16% | 0.5 g |
| Chloroform | 59.4% | 181.7 g |
| Carbon tetrachloride | 0.20% | 0.6 g |
| 1,3-Propylene carbonate | 9.5% | 29.1 g |
| CCl₃C(=O)O(CH₂)₃OH | 27.5% | 84.2 g |
| Others | 3.24% | 10.5 g |

### Example 8

After 262 g (0.99 mol) of hexachloroacetone, 75 g (0.99 mol) of 1,2-propylene glycol, and 4 g of an anion-type ion exchange resin (Amberlyte IRA-900, Cl-form manufactured by Rohm and Haas Company) were charged into a reactor similar to that of Example 1, the temperature was gradually elevated under stirring and a reaction was carried out at an inner temperature of 80°C. While chloroform formed by the reaction was removed by distillation through the distillation line, the reaction was carried out for 3 hours. After the reaction was completed, fractions distilled from the distillation line and a reaction crude liquid present in the reactor were recovered to obtain 322.2 g of a recovered crude liquid (recovery rate: 94.5%). As a result of analysis on GC of an organic component recovered by simple distillation of the recovered crude liquid under vacuum, it was confirmed that compounds shown in Table 8 were formed in yields shown in Table 8.

From the results shown in Table 8, the conversion rate of hexachloroacetone was 100%, the yield of 1,2-propylene carbonate based on hexachloroacetone was 85%, and the yield of chloroform was 92%.

**[Table 8]**

| Compound | GC Composition (% by mass) | Yield |
|---|---|---|
| Hexachloroacetone | 0% | 0 g |
| 1,2-Propylene glycol | 1.6% | 5.2 g |
| Chloroform | 68.7% | 218.6 g |
| Carbon tetrachloride | 0.9% | 3.0 g |
| 1,2-Propylene carbonate | 27.0% | 85.8 g |
| Others | 1.8% | 5.6 g |

### Example 9

After 105.9 g (0.40 mol) of hexachloroacetone and 7.8 g of KF were charged into a 200 mL glass reactor fitted with a stirrer, a dropping funnel, and a distillation line, 49.6 g (0.42 mol) of 3-methylpentanediol was gradually added dropwise at room temperature over a period of 30 minutes. The temperature was gradually elevated under stirring to 120°C over a period of 1 hour. While chloroform formed by the reaction was removed by distillation through the distillation line, the reaction was carried out for 3 hours. Then, the pressure was reduced with maintaining the temperature and the reaction was further carried out for 19 hours. After the reaction was completed, 64 g of a viscous liquid was recovered from the inside of the reactor. After precipitates such as the catalyst were removed by filtration, molecular weight based on polystyrene standards was measured on GPC. The results of the measurement are shown in Table 9. Moreover, 95 g of a distillate was recovered from the distillation line. As a result of analysis on GC of the distillate, it was confirmed that compounds shown in Table 9 were formed in yields shown in Table 9.

**[Table 9]**

| Results of GPC analysis | Mn | Mw |
|---|---|---|
| | 1,521 | 3,048 |

| Results of GC analysis | GC Composition (% by mass) | Yield |
|---|---|---|
| Hexachloroacetone | 0% | 0 g |
| 3-methylpentanediol | 2.2% | 2.1 g |
| Chloroform | 96.8% | 92.0 g |
| Carbon tetrachloride | 0.1% | 0.1 g |
| Others | 0.9% | 0.8 g |

### Example 10

After 19.12 g (0.072 mol) of hexachloroacetone and 0.34 g of KF were charged into a 50 mL glass reactor fitted with a stirrer, a dropping funnel, and a distillation line, 8.67 g (0.115 mol) of 1,3-propanediol was gradually added dropwise at room temperature over a period of 30 minutes. The temperature was gradually elevated under stirring to 120°C over a period of 1 hour. While chloroform formed by the reaction was removed by distillation through the distillation line, the reaction was carried out for 3 hours. Then, the pressure was reduced with maintaining the temperature and the reaction was further carried out for 19 hours. After the reaction was completed, 10.39 g of a viscous liquid was recovered from the inside of the reactor. After precipitates such as the catalyst were removed by filtration, molecular weight based on polystyrene standards was measured on GPC. The results of the measurement are shown in Table 10. Moreover, 17.1 g of a distillate was recovered from the distillation line. As a result of analysis on GC of the distillate, it was confirmed that compounds shown in Table 10 were formed in yields shown in Table 10.

**[Table 10]**

| Results of GPC analysis | Mn | Mw |
|---|---|---|
| | 1,248 | 2,583 |

| Results of GC analysis | GC Composition (% by mass) | Yield |
|---|---|---|
| Hexachloroacetone | 0% | 0 g |
| 1,3-Propanediol | 7.19% | 1.23 g |
| Chloroform | 92.05% | 15.74 g |
| Carbon tetrachloride | 0.58% | 0.1 g |
| Others | 0.18% | 0.03 g |

### Example 11

After 106.1 g (0.401 mol) of hexachloroacetone and 7.8 g of KF were charged into a 200 mL glass reactor fitted with a stirrer, a dropping funnel, and a distillation line, 49.6 g (0.420 mol) of 1,6-hexanediol was gradually added dropwise at room temperature over a period of 30 minutes. The temperature was gradually elevated under stirring to 120°C over a period of 1 hour. While chloroform formed by the reaction was removed by distillation through the distillation line, the reaction was carried out for 3 hours. Then, the pressure was reduced with maintaining the temperature and the reaction was further carried out for 19 hours. After the reaction was completed, 63.1 g of a white solid was recovered from the inside of the reactor. After the solid was heated to a melted state and precipitates such as the catalyst were removed by filtration, molecular weight based on polystyrene standards was measured on GPC. The results of the measurement are shown in Table 11. Moreover, 97 g of a distillate was recovered from the distillation line. As a result of analysis on GC of the distillate, it was confirmed that compounds shown in Table 11 were formed in yields shown in Table 11.

**[Table 11]**

| Results of GPC analysis | Mn | Mw |
|---|---|---|
| | 1,422 | 3,024 |

| Results of GC analysis | GC Composition (% by mass) | Yield |
|---|---|---|
| Hexachloroacetone | 0% | 0 g |
| 1,6-Hexanediol | 4.2% | 4.1 g |
| Chloroform | 94.6% | 91.8 g |
| Carbon tetrachloride | 0.3% | 0.3 g |
| Others | 0.9% | 0.8 g |

### Example 12

After 262 g (0.99 mol) of hexachloroacetone, 91 g (1.98 mol) of ethanol, and 4 g of KF were charged into a 500 mL glass reactor fitted with a stirrer, a reflux condenser at 20°C, and a distillation line, the temperature was gradually elevated under stirring and a reaction was carried out at an inner temperature of 70°C for 10 hours. After the reaction was completed, 355 g of a reaction crude liquid present in the reactor was recovered (recovery rate: 99.5%). As a result of analysis on GC of an organic component recovered by simple distillation of the recovered crude liquid under vacuum, it was confirmed that compounds shown in Table 12 were formed in yields shown in Table 12.

From the results shown in Table 12, the conversion rate of hexachloroacetone was 100%, the yield of diethyl carbonate based on hexachloroacetone was 79%, and the yield of chloroform was 90%.

**[Table 12]**

| Compound | GC Composition (% by mass) | Yield |
|---|---|---|
| Hexachloroacetone | 0% | 0 g |
| Ethanol | 2.6% | 9.0 g |
| Chloroform | 60.4% | 212.1 g |
| Carbon tetrachloride | 0.1% | 0.4 g |
| Diethyl carbonate | 26.2% | 92 g |
| CCl₃C(=O)OCH₂CH₃ | 10.6% | 37.3 g |
| Others | 0.1% | 0.2 g |

### Example 13

After 262 g (0.99 mol) of hexachloroacetone, 257.4 g (1.98 mol) of n-octanol, and 4 g of KF were charged into a reactor similar to that of Example 12, the temperature was gradually elevated under stirring and a reaction was carried out at an inner temperature of 120°C. While chloroform formed by the reaction was removed by distillation through the distillation line, the reaction was carried out for 10 hours. After the reaction was completed, fractions distilled from the distillation line and a reaction crude liquid present in the reactor were recovered to obtain 515.5 g of a recovered crude liquid (recovery rate: 98.5%). As a result of analysis on GC of an organic component recovered by simple distillation of the recovered crude liquid under vacuum, it was confirmed that compounds shown in Table 13 were formed in yields shown in Table 13.

From the results shown in Table 13, the conversion rate of hexachloroacetone was 100%, the yield of dioctyl carbonate based on hexachloroacetone was 88%, and the yield of chloroform was 93%.

**[Table 13]**

| Compound | GC Composition (% by mass) | Yield |
|---|---|---|
| Hexachloroacetone | 0% | 0 g |
| n-Octanol | 2.5% | 13 g |
| Chloroform | 43.2% | 220.9 g |
| Carbon tetrachloride | 0.1% | 0.4 g |
| Dioctyl carbonate | 48.8% | 249.7 g |
| CCl₃C(=O)OC₈H₁₇ | 5.2% | 26.7 g |
| Others | 0.2% | 0.8 g |

### Example 14

After 262 g (0.99 mol) of hexachloroacetone, 186.1 g (1.98 mol) of phenol, and 4 g of KF were charged into a reactor similar to that of Example 12, the temperature was gradually elevated under stirring and a reaction was carried out at an inner temperature of 130°C. While chloroform formed by the reaction was removed by distillation through the distillation line, the reaction was carried out for 30 hours. After the reaction was completed, fractions distilled from the distillation line and a reaction crude liquid present in the reactor were recovered to obtain 450.5 g of a recovered crude liquid (recovery rate: 99.6%). As a result of analysis on GC of an organic component recovered by simple distillation of the recovered crude liquid under vacuum, it was confirmed that compounds shown in Table 14 were formed in yields shown in Table 14.

From the results shown in Table 14, the conversion rate of hexachloroacetone was 100%, the yield of diphenyl carbonate based on hexachloroacetone was 0.99%, and the yield of chloroform was 50%.

**[Table 14]**

| Compound | GC Composition (% by mass) | Yield |
|---|---|---|
| Hexachloroacetone | 0% | 0 g |
| Phenol | 21.0% | 93.7 g |
| Chloroform | 26.2% | 117.2 g |
| Carbon tetrachloride | 0.09% | 0.4 g |
| Diphenyl carbonate | 0.47% | 2.1 g |
| CCl₃C(=O)OC₆H₅ | 51.6% | 230.3 g |
| Others | 0.64% | 2.8 g |

### Example 15

After 262 g (0.99 mol) of hexachloroacetone, 91 g (1.98 mol) of ethanol, 2 g of KF, and 2 g of cerium oxide (CeO/Ce₂O₃: manufactured by Daiichi Kigenso Kagaku Kogyo Co., Ltd.) were charged into a reactor similar to that of Example 12, the temperature was gradually elevated under stirring and a reaction was carried out at an inner temperature of 70°C for 10 hours. After the reaction was completed, 355 g of a recovered crude liquid present in the reactor was recovered (recovery rate: 99.5%). As a result of analysis on GC of an organic component recovered by simple distillation of the recovered crude liquid under vacuum, it was confirmed that compounds shown in Table 15 were formed in yields shown in Table 15.

From the results shown in Table 15, the conversion rate of hexachloroacetone was 100%, the yield of diethyl carbonate based on hexachloroacetone was 99%, and the yield of chloroform was 99%.

**[Table 15]**

| Compound | GC Composition (% by mass) | Yield |
|---|---|---|
| Hexachloroacetone | 0% | 0 g |
| Ethanol | 0% | 0 g |
| Chloroform | 67.0% | 235.0 g |
| Carbon tetrachloride | 0.05% | 0.2 g |
| Diethyl carbonate | 32.9% | 115.6 g |
| CCl₃C(=O)OCH₂CH₃ | 0% | 0 g |
| Others | 0.05% | 0.2 g |

### Example 16

After 262 g (0.99 mol) of hexachloroacetone, 91 g (1.98 mol) of ethanol, 2 g of KF, 2 g of KF, and 2 g of zirconia (ZrO₂, manufactured by Daiichi Kigenso Kagaku Kogyo Co., Ltd.) were charged into a 500 mL pressure tight reactor made of Hastelloy, the temperature was gradually elevated under stirring and a reaction was carried out at an inner temperature of 140°C for 10 hours. After the reaction was completed, 356 g of a recovered crude liquid present in the reactor was recovered (recovery rate: 99.7%). As a result of analysis on GC of an organic component recovered by simple distillation of the recovered crude liquid under vacuum, it was confirmed that compounds shown in Table 16 were formed in yields shown in Table 16.

From the results shown in Table 16, the conversion rate of hexachloroacetone was 100%, the yield of diethyl carbonate based on hexachloroacetone was 99.4%, and the yield of chloroform was 99.5%.

**[Table 16]**

| Compound | GC Composition (% by mass) | Yield |
|---|---|---|
| Hexachloroacetone | 0% | 0 g |
| Ethanol | 0% | 0 g |
| Chloroform | 66.9% | 235.5 g |
| Carbon tetrachloride | 0.05% | 0.2 g |
| Diethyl carbonate | 33.0% | 116.1 g |
| CCl₃C(=O)OCH₂CH₃ | 0% | 0 g |
| Others | 0.05% | 0.2 g |

### Example 17

Into a 500 mL glass reactor fitted with a stirrer, a reflux condenser at 20°C, and a distillation line was charged 4 g of NaH, and then 91 g (1.98 mol) of ethanol was gradually added dropwise over a period of 30 minutes. After completion of the dropwise addition, 262 g (0.99 mol) of hexachloroacetone was added dropwise under cooling with a water bath so that the inner temperature did not reach 50°C or higher. After completion of the dropwise addition, the temperature was gradually elevated under stirring and a reaction was carried out at an inner temperature of 70°C for 10 hours. After the reaction was completed, 350 g of a recovered crude liquid present in the reactor was recovered (recovery rate: 98.0%). As a result of analysis on GC of an organic component recovered by simple distillation of the recovered crude liquid under vacuum, it was confirmed that compounds shown in Table 17 were formed in yields shown in Table 17.

From the results shown in Table 17, the conversion rate of hexachloroacetone was 100%, the yield of diethyl carbonate based on hexachloroacetone was 0%, and the yield of chloroform was 46.6%.

**[Table 17]**

| Compound | GC Composition (% by mass) | Yield |
|---|---|---|
| Hexachloroacetone | 0% | 0 g |
| Ethanol | 12.1% | 40.7 g |
| Chloroform | 32.9% | 110.2 g |
| Carbon tetrachloride | 10.06% | 0.2 g |
| Diethyl carbonate | 0% | 0 g |
| CCl₃C(=O)OCH₂CH₃ | 53.1% | 177.9 g |
| | 11.84% | 6.2 g |

### Example 18

Into a 500 mL glass reactor fitted with a stirrer, a reflux condenser at 20°C, and a distillation line was charged 4 g of Na, and then 91 g (1.98 mol) of ethanol was gradually added dropwise over a period of 30 minutes. After completion of the dropwise addition, 262 g (0.99 mol) of hexachloroacetone was added dropwise under cooling with a water bath so that the inner temperature did not reach 50°C or higher. After completion of the dropwise addition, the temperature was gradually elevated under stirring and a reaction was carried out at an inner temperature of 70°C for 10 hours. After the reaction was completed, 353 g of a recovered crude liquid present in the reactor was recovered (recovery rate: 98.9%). As a result of analysis on GC of an organic component recovered by simple distillation of the recovered crude liquid under vacuum, it was confirmed that compounds shown in Table 18 were formed in yields shown in Table 18.

From the results shown in Table 18, the conversion rate of hexachloroacetone was 100%, the yield of diethyl carbonate based on hexachloroacetone was 0%, and the yield of chloroform was 46.6%.

**[Table 18]**

| Compound | GC Composition (% by mass) | Yield |
|---|---|---|
| Hexachloroacetone | 0% | 0 g |
| Ethanol | 12.1% | 40.7 g |
| Chloroform | 32.9% | 110.8 g |
| Carbon tetrachloride | 0.06% | 0.2 g |
| Diethyl carbonate | 0% | 0 g |
| CCl₃C(=O)OCH₂CH₃ | 52.7% | 177.5 g |
| Others | 2.24% | 7.3 g |

This application is based on Japanese Patent Application No. 2007-312655 filed December 3, 2007, Japanese Patent Application No. 2007-321773 filed December 13, 2007 and Japanese Patent Application No. 2008-208726 filed August 13, 2008.

### INDUSTRIAL APPLICABILITY

The dialkyl carbonates and diaryl carbonates obtained by the production process of the invention can be applied to various uses and are useful as organic solvents, resin raw materials, raw materials for pharmaceuticals and agricultural chemicals, and the like. Also, the diaryl carbonates are also useful as heat-resistant media.

Moreover, the cyclic carbonates obtained by the production process of the invention are industrially extremely useful as solvent applicable for various uses, electrolytes, resist removers, acrylic fiber processors, hydroxyethylating agents, raw materials for pharmaceuticals, soil hardeners, and the like.

Furthermore, the polycarbonates obtained by the production process of the invention are useful, as oligomers having a reactive OH group in the terminal, as raw materials for various polymer materials such as highly functional polyurethanes, polyesters, polycarbonates, and epoxy resins, reactive diluents, reactive plasticizers, and the like.

## Claims

1. A process for producing a carbonate compound comprising reacting a compound represented by the following formula (1) with a compound having one OH group or a compound having two or more OH groups in the presence of a catalyst to obtain a compound having a carbonate bond,
wherein the catalyst comprises a halogen salt: 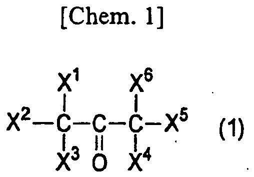 wherein X¹ to X⁶ each represents a chlorine atom.

2. The process for producing a carbonate compound according to claim 1, wherein the halogen salt comprises one or more member selected from the group consisting of halogen salts of alkali metals, halogen salts of alkali earth metals, halogen salts of ammoniums, halogen salts of quaternary ammoniums, and ionexchange resins having a halogen salt structure.

3. The process for producing a carbonate compound according to claim 1 or 2, wherein the halogen salt is a fluoride of an alkali metal.

4. The process for producing a carbonate compound according to claim 1 or 2, wherein the halogen salt is a quaternary ammonium bromide.

5. The process for producing a carbonate compound according to any one of claims 1 to 4, wherein the reaction is carried out in the presence of the catalyst and a promoter, wherein the promoter is a solid acid catalyst.

6. The process for producing a carbonate compound according to claim 5, wherein the solid acid catalyst comprises at least one member selected from the group consisting of metal oxides having a strong acid point, heteropoly acids, and cation-exchange resins.

7. The process for producing a carbonate compound according to claim 6, wherein the metal oxides having a strong acid point comprises at least one member selected from the group consisting of cerium oxide (CeO₂/Ce₂O₃), silica-alumina (SiO₂·Al₂O₃), γ-alumina (Al₂O₃), silica-magnesia (SiO₂·MgO), zirconia (ZrO₂), silica-zirconia (SiO₂·ZrO₂), ZnO·ZrO₂, and Al₂O₃·B₂O₃.

8. The process for producing a carbonate compound according to any one of claims 1 to 7, wherein the compound having a carbonate bond is a compound represented by the following formula (31) or a compound represented by the following formula (32): 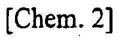 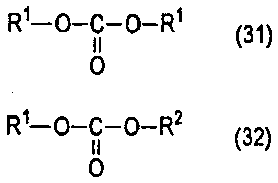 wherein R¹ and R² each represents a monovalent aliphatic hydrocarbon group or a monovalent aromatic hydrocarbon group, provided that R¹ and R² are not the same group.

9. The process for producing a carbonate compound according to any one of claims 1 to 7, wherein the compound having a carbonate bond is a cyclic carbonate compound represented by the following formula (3a): 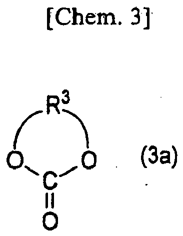 wherein R³ represents a divalent aliphatic hydrocarbon group or a divalent aromatic hydrocarbon group.

10. The process for producing a carbonate compound according to any one of claims 1 to 7, wherein the compound having a carbonate bond is a linear carbonate compound represented by the following formula (3b): 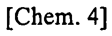 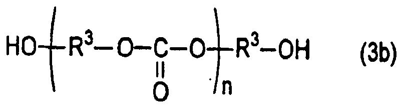 wherein R³ represents a divalent aliphatic hydrocarbon group or a divalent aromatic hydrocarbon group.

11. The process for producing a carbonate compound according to any one of claims 1 to 8, wherein the compound having one OH group comprises at least one member selected from the group consisting of methanol, ethanol, n-propanol, i-propanol, n-butanol, t-butanol, 3-oxa-n-butanol, and phenol.

12. The process for producing a carbonate compound according to any one of claims 1 to 7, 9 and 10, wherein the compound having two or more OH groups comprises at least one member selected from the group consisting of ethylene glycol, 1,2-propylene glycol, 3-methyl-1,5-pentanediol, 3-oxa-1,5-pentanediol, 1,6-hexanediol, 1,3-propanediol, 1,2-butanediol, and 1,4-butanediol.

## Patentansprüche

1. Verfahren zur Herstellung einer Carbonatverbindung, umfassend das Umsetzen einer Verbindung, dargestellt durch die folgende Formel (1), mit einer Verbindung mit einer OH-Gruppe oder einer Verbindung mit zwei oder mehreren OH-Gruppen in der Gegenwart eines Katalysators, um eine Verbindung mit einer Carbonatbindung zu erhalten,
wobei der Katalysator ein Halogenidsalz umfasst: 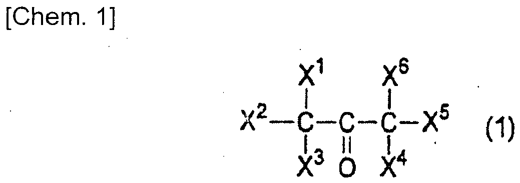 wobei jedes von X¹ bis X⁶ ein Chloratom darstellt.

2. Verfahren zur Herstellung einer Carbonatverbindung nach Anspruch 1, wobei das Halogenidsalz einen oder mehrere Vertreter, ausgewählt aus der Gruppe, bestehend aus Alkalimetallhalogenidsalzen, Erdalkalimetallhalogenidsalzen, Ammoniumhalogenidsalzen, quaternären Ammoniumhalogenidsalzen und Ionenaustauscherharzen mit einer Halogenidsalzstruktur, umfasst.

3. Verfahren zur Herstellung einer Carbonatverbindung nach Anspruch 1 oder 2, wobei das Halogenidsalz ein Alkalimetallfluorid ist.

4. Verfahren zur Herstellung einer Carbonatverbindung nach Anspruch 1 oder 2, wobei das Halogenidsalz ein quaternäres Ammoniumbromid ist.

5. Verfahren zur Herstellung einer Carbonatverbindung nach einem der Ansprüche 1 bis 4, wobei die Umsetzung in der Gegenwart des Katalysators und eines Promotors durchgeführt wird, wobei der Promotor ein saurer Festphasenkataly-sator ist.

6. Verfahren zur Herstellung einer Carbonatverbindung nach Anspruch 5, wobei der saure Festphasenkatalysator mindestens einen Vertreter, ausgewählt aus der Gruppe, bestehend aus Metalloxiden mit einem starken Säurepunkt, Heteropolysäuren und Kationenaustauscherharzen, umfasst.

7. Verfahren zur Herstellung einer Carbonatverbindung nach Anspruch 6, wobei das Metalloxid mit einem starken Säurepunkt mindestens einen Vertreter, ausgewählt aus der Gruppe, bestehend aus Ceroxid (CeO₂/Ce₂O₃), Siliciumdioxid-Aluminiumoxid (SiO₂·Al₂O₃), γ-Aluminiumoxid (Al₂O₃), Siliciumdioxid-Magnesiumoxid (SiO₂·MgO), Zirconiumdioxid (ZrO₂), Siliciumdioxid-Zirconiumdioxid (SiO₂·ZrO₂), ZnO·ZrO₂ und Al₂O₃·B₂O₃, umfasst.

8. Verfahren zur Herstellung einer Carbonatverbindung nach einem der Ansprüche 1 bis 7, wobei die Verbindung mit einer Carbonatbindung eine Verbindung, dargestellt durch die folgende Formel (31), oder eine Verbindung, dargestellt durch die folgende Formel (32), ist: 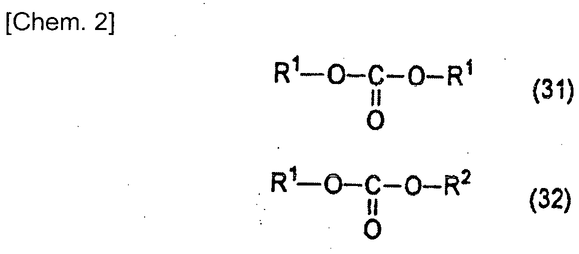 wobei jedes von R¹ und R² eine einwertige aliphatische Kohlenwasserstoffgruppe oder eine einwertige aromatische Kohlenwasserstoffgruppe darstellt, mit der Maßgabe, dass R¹ und R² nicht die gleiche Gruppe sind.

9. Verfahren zur Herstellung einer Carbonatverbindung nach einem der Ansprüche 1 bis 7, wobei die Verbindung mit einer Carbonatbindung eine cyclische Carbonatverbindung, dargestellt durch die folgende Formel (3a), ist: 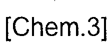 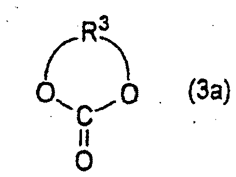 wobei R³ eine zweiwertige aliphatische Kohlenwasserstoffgruppe oder eine zweiwertige aromatische Kohlenwasserstoffgruppe darstellt.

10. Verfahren zur Herstellung einer Carbonatverbindung nach einem der Ansprüche 1 bis 7, wobei die Verbindung mit einer Carbonatbindung eine lineare Carbonatverbindung, dargestellt durch die folgende Formel (3b), ist: 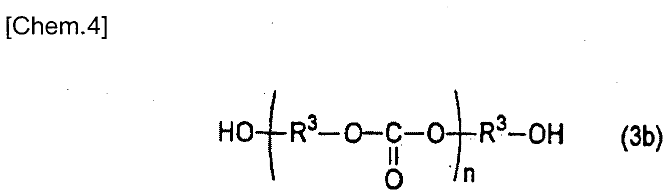 wobei R³ eine zweiwertige aliphatische Kohlenwasserstoffgruppe oder eine zweiwertige aromatische Kohlenwasserstoffgruppe darstellt.

11. Verfahren zur Herstellung einer Carbonatverbindung nach einem der Ansprüche 1 bis 8, wobei die Verbindung mit einer OH-Gruppe mindestens einen Vertreter, ausgewählt aus der Gruppe, bestehend aus Methanol, Ethanol, n-Propanol, i-Propanol, n-Butanol, t-Butanol, 3-Oxa-n-butanol und Phenol, umfasst.

12. Verfahren zur Herstellung einer Carbonatverbindung nach einem der Ansprüche 1 bis 7, 9 und 10, wobei die Verbindung mit zwei oder mehreren OH-Gruppen mindestens einen Vertreter, ausgewählt aus der Gruppe, bestehend aus Ethylenglycol, 1,2-Propylenglycol, 3-Methyl-1,5-pentandiol, 3-Oxa-1,5-pentandiol, 1,6-Hexandiol, 1,3-Propandiol, 1,2-Butandiol und 1,4-Butandiol, umfasst.

## Revendications

1. Procédé de production d'un composé carbonate comprenant la réaction d'un composé représenté par la formule (1) suivante avec un composé ayant un radical OH ou un composé ayant deux radicaux OH ou plus, en présence d'un catalyseur pour obtenir un composé ayant une liaison carbonate,
où le catalyseur comprend un sel d'halogène : 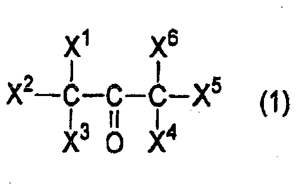 où X₁ à X⁶ représentent chacun, l'atome de chlore.

2. Procédé de production d'un composé carbonate selon la revendication 1, où le sel d'halogène comprend un ou plusieurs membres choisis parmi le groupe consistant en des sels d'halogène de métaux alcalins, des sels d'halogène de métaux alcalino-terreux, des sels d'halogène d'ammoniums, des sels d'halogènes d'ammoniums quaternaires, et des résines échangeuses d'ions ayant une structure de sel d'halogène.

3. Procédé de production d'un composé carbonate selon la revendication 1 ou 2, où le sel d'halogène est un fluorure d'un métal alcalin.

4. Procédé de production d'un composé carbonate selon la revendication 1 ou 2, où le sel d'halogène est un bromure d'ammonium quaternaire.

5. Procédé de production d'un composé carbonate selon l'une quelconque des revendications 1 à 4, où la réaction est réalisée en présence du catalyseur et d'un promoteur, où le promoteur est un catalyseur acide solide.

6. Procédé de production d'un composé carbonate selon la revendication 5, où le catalyseur acide solide comprend au moins un membre choisi parmi le groupe consistant en des oxydes métalliques ayant un point d'acide fort, des hétéropolyacides et des résines échangeuses de cations.

7. Procédé de production d'un composé carbonate selon la revendication 6, où les oxydes métalliques ayant un point d'acide fort comprennent au moins un membre choisi parmi le groupe consistant en l'oxyde de cérium (CeO₂/Ce₂O₃), silice-alumine (SiO₂·Al₃O₃), la γ-alumine (Al₂O₃), la silice-magnésie (SiO₂·MgO), l'oxyde de zirconium (ZrO₂), la silice-zircone (SiO₂. ZrO₂), ZnO.ZrO₂ et Al₂O₃.B₂O₃.

8. Procédé de production d'un composé carbonate selon l'une quelconque des revendications 1 à 7, où le composé ayant une liaison carbonate est un composé représenté par la formule (31) suivante ou un composé représenté par la formule (32) suivante : 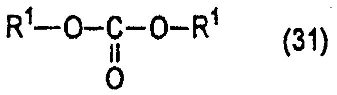 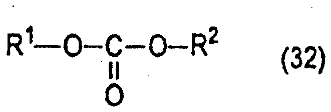 où R¹ et R² représentent chacun, un radical hydrocarboné aliphatique monovalent ou un radical hydrocarboné aromatique monovalent, pourvu que R¹ et R² ne sont pas le même radical.

9. Procédé de production d'un composé carbonate selon l'une quelconque des revendications 1 à 7, où le composé ayant une liaison carbonate est un composé carbonate cyclique représenté par la formule (3a) suivante : 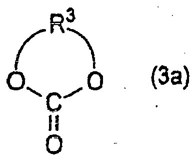 où R³ représente un radical hydrocarboné aliphatique bivalent ou un radical hydrocarboné aromatique bivalent.

10. Procédé de production d'un composé carbonate selon l'une quelconque des revendications 1 à 7, où le composé ayant une liaison carbonate est un composé carbonate linéaire représenté par la formule (3b) suivante : 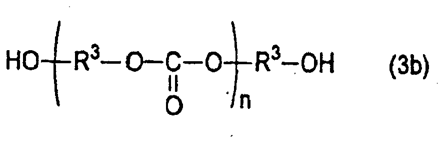 où R³ représente un radical hydrocarboné aliphatique bivalent ou un radical hydrocarboné aromatique bivalent.

11. Procédé de production d'un composé carbonate selon l'une quelconque des revendications 1 à 8, où le composé ayant un radical OH comprend au moins un membre choisi parmi le groupe consistant en le méthanol, l'éthanol, le n-propanol, l'i-propanol, le n-butanol, le t-butanol, le 3-oxa-n-butanol et le phénol.

12. Procédé de production d'un composé carbonate selon l'une quelconque des revendications 1 à 7, 9 et 10, où le composé ayant deux radicaux OH ou plus comprend au moins un membre choisi parmi le groupe consistant en l'éthylèneglycol, le 1,2-propylèneglycol, le 3-méthyl-1,5-pentanediol, le 3-oxa-1,5-pentanediol, le 1,6-hexanediol, le 1,3-propanediol, le 1,2-butanediol et le 1,4-butanediol.
